# EUROPEAN PATENT APPLICATION

(11) **EP 2 088 199 A1**
(43) Date of publication of application: **12.08.2009**
(21) Application number: 09000696.6
(22) Date of filing: 20.01.2009
(51) Int. Cl.: C12N 15/52, C12N 15/70, C12P 23/00

(54) **A strain of genetically reengineered escherichia coli for biosynthesis of high yield carotenoids after mutation screening**

(30) Priority: 05.02.2008 TW 97100460
(71) Applicant: Echem Hightech Co., Ltd., Hu-Kou Hsieng Hsin chu 303 (TW)
(72) Inventor: Chiou, Ming-Hsi, 303 Hsin-Chu Hsien (TW); Wu, Mei-Chiao, 303 Hsin-Chu Hsien (TW); Yang, Ming-Haw, 303 Hsin-Chu Hsien (TW); Lin, Chi-Nan, 303 Hsin-Chu Hsien (TW); Lu, Yi-Chen, 303 Hsin-Chu Hsien (TW)
(74) Representative: Winkler, Andreas Fritz Ernst

(57) **Abstract**

The present invention relates to a strain of *Escherichia coli* (*E. coli* M2H) for biosynthesis of high yield carotenoids, the steps for obtaining *E. coli* M2H comprises: purifying the host strain JCL1613 for pCW9/P2IDI; create plasmids for producing carotenoid genes; transform the plasmids into JCL1613; utilizing physical method (UV) to induce and improve JCL1613; select the portions with 99% death rate; utilizing 50mL shake flask incubation; and go through UV screening to obtain the mutated strain of *E. coli* M2H.

## Description

### BACKGROUND OF THE INVENTION

### 1. Field of the Invention

The present invention relates to a process of producing high content natural carotenoids, especially relating to *Escherichia coli* (*E. coli*) that may produce high content carotenoids through genetically engineered modifications, and its high cell density fermentation process and recovery and purification method.

### 2. Description of the Prior Art

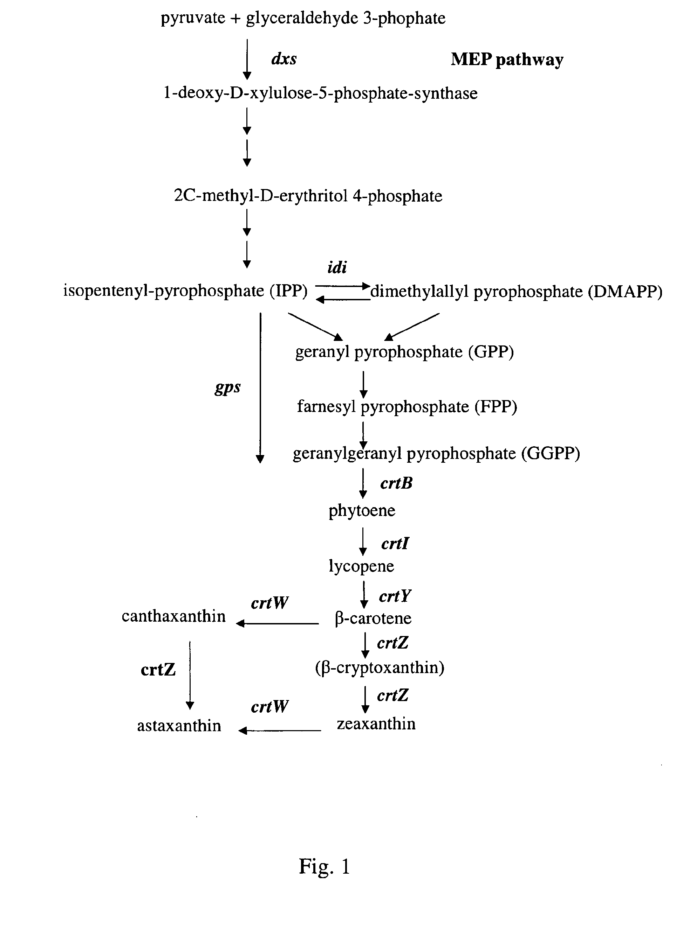

Utilizing genetic engineering technology to generate exogenous proteins as medical purpose proteins is a well known process in biotechnology, however, other than proteins, there are many other secondary metabolites that are essential to the human body, such as vitamins etc.. Carotenoid is a natural pigment that may be applied to feed additives, food additives, cosmetic manufacturing etc. and is abundant in a number of common plants and microorganisms, at the present, more than 600 carotenoids have been identified. With the advances and analysis in molecular biology, various metabolic pathways for carotenoids are continually being discovered, as shown in FIG. 1. Within these varieties of carotenoids, only a few may be chemically synthesized for commercial production, and some may be extracted from natural sources or obtained through fermentation (P. C. Lee, 2002), thus, utilizing biotechnology to produce these types of chemical compounds is a target that scientists are hoping to achieve.

Lycopene is the first metabolite to form colors in the previously mentioned metabolic paths of carotenoids, the synthesis path may be formed from pyruvate and glyceraldehyde-3-phosphate (G-3-P) after a series of reduction, dehydration, phosphorylation and other biochemical reactions. It can be further catalyzed into β-carotene, zeaxanthin, β-cryptoxanthin, canthaxanthin and astaxanthin etc. (refer to FIG. 1) through cyclic reaction catalyzed by different cyclases. Lycopene have been recognized as an industrially important natural food coloring agent due to its high safety and high staining power in the red region. Recently, lycopene has been proved to be a strong antioxidant which neutralizes free radicals, especially those derived from oxygen and also has the ability of inhibiting LDL (low-density lipoprotein) oxidation. This will result in reducing cholesterol levels in the blood and prevent the body from the attacks of free radicals.

Clinic researches have shown that lycopene has the potency to protect against prostate cancer, breast cancer and cardiovascular disease. In addition, other preliminary researches also suggest that lycopene may reduce the risk of macular degeneration, serum lipid oxidation and cancers of the lung, bladder, cervix and skin. Some of the chemical properties and effects of lycopene mentioned above may be referred to the publications by Giovannucci et al. in J. Natl. Cancer Inst. 87(23):1767-1776, Dec. 6, 1995, and Morris et al. in J. Amer. Med. Assoc. 272(18):1439-1441, 1994.

The existing productions of lycopene are carried out via chemical synthesis (produced by BASF and DSM), where lycopene is produced through the extraction of tomatoes and fermentation of Blakeslea trispora (refer to U.S. Pat. No. 3,097,146 and 3,369,974), on the other hand, productions via biotechnology are still mainly at the research and development stage, for instance, in 2006, Yoon et al. utilized the recombination of *E. coli* to produce lycopene, and have achieved the content of 102mg/L in 72 hours. In 2005, Kang et al. utilized the common performance of other related genes, and have achieved the content of 4.7mg/g under shake flask incubation. In 2007, Jin YS also employed the genes' common performance and destruction method to achieve a lycopene yield of 16mg/g under shake flask incubation.

In order to raise the lycopene yield, the research conducted by W. R. Farmer et al. pointed out that the precursor of carotenoids, isopentenyl pyrophosphate (IPP) is composed of pyruvate and glyceraldehydes-3-phosphate (G-3-P), thus, the critical gene (pykFA) associated with the reverse reaction of glycolysis in the *E. coli* chromosome DNA has been blocked to increase the pyruvate and glyceraldehydes-3-phosphate (G-3-P) contents, also raising the IPP content and therefore increase the yield for carotenoids (as published by W. R., Farmer and J. C., Liao in Biotechnol. Prog. Vol. 17 Pg. 57-61, 2001).

In addition, if bacteria are used to mass represent exogenous genes, it would usually result in the imbalance of metabolism, thus cause the feedback inhibition mechanism. In order to prevent the activation of feedback inhibition mechanism, J.C. Liao has utilized the saccharide metabolism (acetyl phosphate, ACP), NRI (glnG product) and glnAP2 promoter to form a dynamic control ring to further control idi (isopentenyl diphosphate isomerase, isoenzyme of IPP) genes, thus raising the lycopene yield ( U.S. Pat. No. 7,122,341 and as published by W. R., Farmer and J. C., Liao in Nature Biotechnology Vol. 18 Pg. 533-537, 2000).

According to the biosynthetic pathway of carotenoids, once lycopene is catalyzed by lycopene cyclase enzymes (crtY) and β-carotene hydroxylase (or crtZ), it may be biosynthesized into zeaxanthin. In the daily diet, zeaxanthins are mainly found in cabbages, spinaches, leaf mustards, corns and other vegetables (as published by AR. Mangels et al. in J, Am. Diet Assoc. Vol. 93 Pg. 284-296, 1993), it cannot be synthesized by the human body and needs to be assimilated from foods. Researches have shown that zeaxanthin can be found within the milk and blood of the human body (as published by F. Khachik et al. in Investig. Ophthamol. Vis. Sci. Vol. 38 Pg. 1802-1811, 1997), thus, the body needs to acquire zeaxanthin via food assimilation. The main physiological activities of zeaxanthin are centralized at the cornea region and the eye cells to absorb the anti-oxidation effect of free radicals. In addition, it clearly offers protection against age-related macular degeneration (AMD) caused by long term ultraviolet (UV) rays and photolethal effects, and against cataracts caused by aging of eye lens (as published by S. Beatty, J. Nolan et al. in Arch. Biochem Biophys Vol. 430 Pg. 70-76, 2004).

Currently, methods for zeaxanthin production are categorized into: fermentation of natural microorganisms, plant extraction, and chemical synthesis. The production of zeaxanthin via fermentation of natural microorganisms has only been used on microorganisms such as *Flavobacterium multivorum* (U.S. Pat. No. 5,427,783, RE38009E, and 6,291,204), *Neospongiococcum excentrium* (U.S. Pat. No. 5,360,730) and *Paracoccus* (U.S. Pat. No. 5,935,808) etc.. The major sources of plant extraction are marigold, alfalfa and Lycium Chinese Mill (LCM) berries (U.S. Pat. No. 7,173,745 and 7,109,360). Chemical synthesis methods for zeaxanthin production may be referred to U.S. Pat. No. 6,818,798, 6,747,177 and 6,743,954.

During the biosynthesis process of zeaxanthin, the resulting product of β-cryptoxanthin may be obtained via the appropriate truncation of N-terminal amino acid sequences in the β-carotene hydroxylase (as published by Zairen Sun et al. in J. Biol. Chem. Vol. 271 No. 40 Pg. 24349-24353, 1996). β-cryptoxanthin is a type of natural carotenoid commonly contained in yellow or orange vegetables and fruits, such as citruses, mangoes, kiwifruits etc., wherein higher contents of β-cryptoxanthin are found in citrus peels. β-cryptoxanthin not only provides coloring effects and anti-oxidation, it can also be used as the precursor for vitamin A. In addition, recent academic researches have shown that β-cryptoxanthin may lower the risk for developing arthritis.

Due to the previously mentioned beneficial efficacies of β-cryptoxanthin toward the human body, it has become a striving goal for the industry to find a quick and effective way to obtain the substance. The major sources and production methods of β-cryptoxanthin include: (1) chemical synthesis production, such as U.S. Pat. No. 7,115,786, where a method of utilizing lutein as the initial reagent to obtain β-cryptoxanthin and alpha-cryptoxanthin has been disclosed; (2) via the extraction of existing plants, such as the method described by U.S. Pat. No. 6,262,284, where a small amount of β-cryptoxanthin and a considerable quantity of zeaxanthin may be obtained through the extraction of LCM berries; (3) via the fermentation of microorganisms, such as the fermentation production method utilizing natural microorganisms described in U.S. Pat. No. 5,935,808.

From the biosynthetic pathway of carotenoids, it is known that β-carotene may be biosynthesized into canthaxanthin when β-carotene is catalyzed by β-carotene ketolases (crtW). Usually, canthaxanthin exists naturally in fungi, crustaceans, fishes, green alga and eggs. Canthaxanthins are mainly used as additives for animal feeds, giving salmons, egg yolks and poultry products a light red and brighter color; it may also be applied to jams, candies, syrups, sauces, carbonated drinks and other foods as coloring agent. Canthaxanthins are more commonly used as animal feeds, rather than coloring agents. In fact, salmon is slightly pink in color because it eats shrimps for food, the market acceptance rate would be higher if farmed salmons are able to display the same color, which explains why canthaxanthins are used as food additives; the same goes to poultries, as the skins and egg yolks of poultries may display a brilliant yellow color with the use of canthaxanthins. Using this type of additive does not affect the quality of foods but only change the colors of foods. Canthaxanthin is also found in common plants or microorganisms as a type of photochemical, at present, the major production method for canthaxanthin is production through chemical synthesis. Fermentation production examples utilizing microorganisms have also been documented (Prakash Bhosale, 2005), however, the yield is too low and does not meet the requirements for commercial production.

Astaxanthin is the end product in the biosynthetic pathway of carotenoids, it may be formed via the transformation of canthaxanthin with the help of β-carotene hydroxylase (crtZ), or it can be formed via the transformation of zeaxanthin with the help of β-carotene ketolases (crtW). Astaxanthin generally exists in animals (birds or fishes), plants, alga and microorganisms (especially yeasts), it is a natural coloring and also a very strong anti-oxidant, with an anti-oxidation ability 500 times than that of vitamin E. Astaxanthins' ability to eliminate reactive oxygen free radicals may be expected to be applied for medical purposes, to treat certain illnesses, such as cancer. At present, astaxanthins are mainly used as coloring agents in industrial applications, by adding astaxanthins into feeds for the cultivation of salmons and trout, red and flavorful meat may be obtained within a short period of time. Also, the anti-oxidation characteristic of astaxanthins is also widely applied to skin care and cosmetic products.

There are mainly three types of production methods for astaxanthin: extraction method, chemical synthesis method (U.S. Pat. No. 2005/0214897) and fermentation method. Extraction methods are mainly applied to crustaceans and flowers of plants (Adonis aestivalis, refer to U.S. Pat. No. 5,453,565). Yeasts and alga are used to produce astaxanthins in most fermentation methods, the most widely used yeast is usually improved strains of *Phaffia Rhodozyma* or changing the nutrient medium to improve its yield (Taiwan Pat. No. 83100549 and U.S. Pat. No. 5,356,809), U.S. Pat. No. 2005/0124032 utilizes *Xanthophyllomyces Dendrorhous* to produce astaxanthin, its yield may reach 425mg/L. On the other hand, green alga (Chlorella zofingiensis) may also be used for the fermentation production of astaxanthin (U.S. Pat. No. 2005/0214897). If red alga Haematococcus (U.S. Pat. No. 6,022,701) is used for the production, light source and a longer cultivation time would be required. The production methods mentioned above are not only time consuming but would also result in low yields.

From the above, it can be seen that at present, the key methods for producing carotenoid products include chemical synthesis, natural extraction and fermentation, each with their own problems and shortcomings. For example, chemical synthesis may be difficult to synthesize due to the complexity of carotenoid structures, or chemical synthesis may produce some isomers that do not exist in nature. If natural extraction is employed, it will be limited by the low content and purity of the original material; if fermentation is used, it would usually be limited to producing one single product per bacterium.

In view of the above, the present invention is based on metabolic control (U.S. Pat. No. 7,122,341), where a strain is obtained through classical and genetic engineering strain improvement, it may produce high yield carotenoid compounds under the existence of carotenoid biosynthetic genes and regulatory genes, and via high cell density fermentation, in addition, these compounds include lycopene, zeaxanthin, β-cryptoxanthin, canthaxanthin and astaxanthin.

### SUMMARY OF THE INVENTION

The present invention is mainly based on the metabolic engineering control method suggested by Liao et al. (2000), and further performs strain improvement to obtain a higher lycopene yield production strain, at the same time, based on the biosynthetic pathways, further constructs production strains for other compounds, such as zeaxanthin, β-cryptoxanthin, canthaxanthin and astaxanthin etc. with the assistance of high cell density fermentation technology, and recovery purification technology. The present invention has already dealt with the bottleneck problem of producing carotenoids with biotechnology, and has reached the goal of commercial production. The present invention provides a mode of production utilizing biological cell factories, and techniques involved with respect to a series of products will be described in detail below, in order to show the technical characteristics of the present invention.

The present invention will describe in detail some preferred embodiments. However, it should be noted that other than these detailed descriptions, the present invention may be practiced in a wider range of other embodiments besides those explicitly described, and the scope of the present invention is not expressly limited except as specified in the accompanying claims.

The present invention provides a biological cell platform for diversified production of large quantities of carotenoids, which, along with high cell density fermentation technology, will be able to produce high yield and high purity carotenoid products within a short period.

The object of the present invention is to provide a process of carotenoid production, which, through genetically engineered modification of *Escherichia coli* (*E. coli*) may produce high content carotenoids.

Another object of the present invention is to provide a process of carotenoid production, which provides the high cell density fermentation process of *E. coli* for producing carotenoids.

Yet another object of the present invention is to provide a process of carotenoid production, which provides the recovery purification method for carotenoids.

The present invention is related to a process for the creation of genetically engineered bacteria, and in coordination with high cell density fermentation for the rapid mass production to attain high purity through recovery purification.

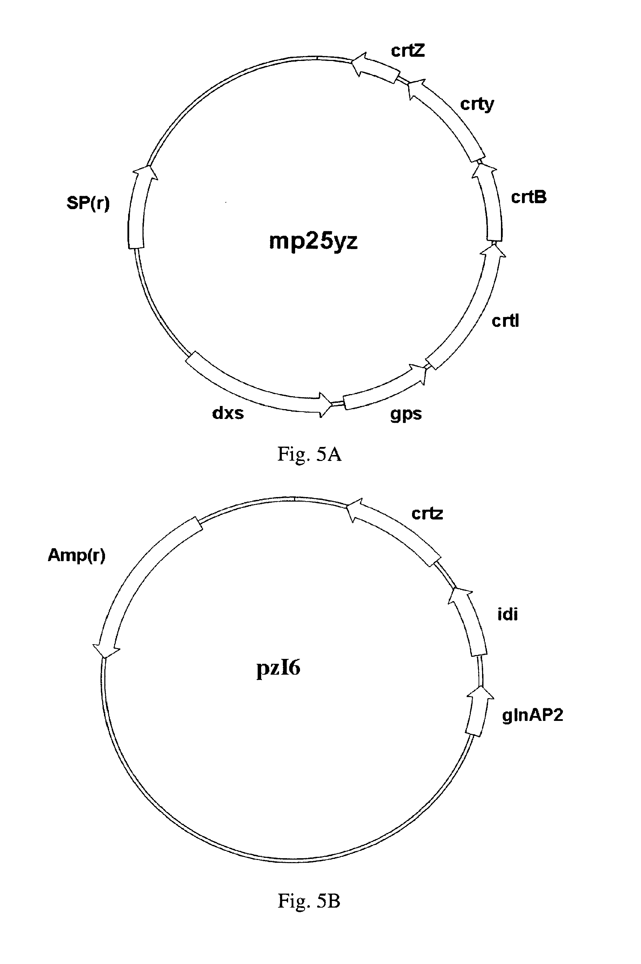

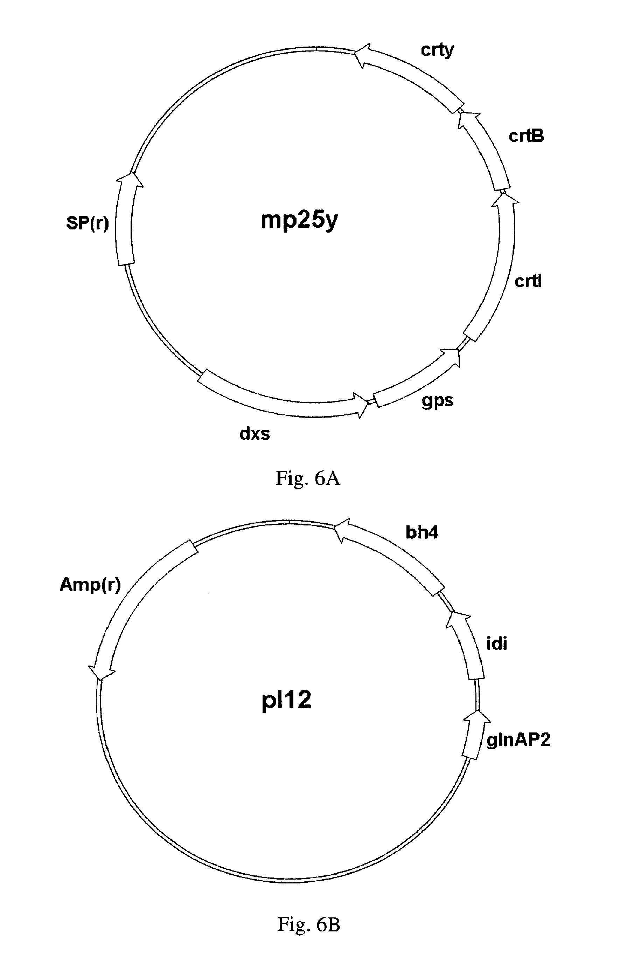

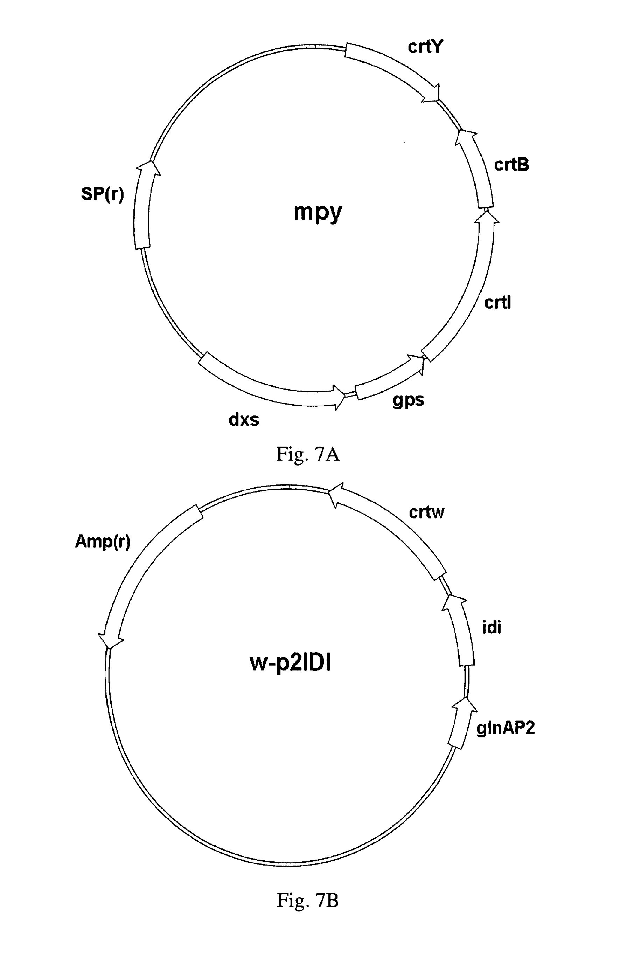

The process described by the present invention involves the following steps:
(a) host screening for the genetically engineered bacteria: using lycopene as the index, screen for a host with a high yield of carotenoids. First, JCL1613 (host for BCRC940321) is used as the host to produce lycopene; the genes for producing lycopene, including dxs, gps, crtB and crtI are created in plasmid PCL1920, this particular plasmid carrying multiple genes is named mp25, while the plasmid carrying gene idi and promoter glnAP2 is named p2IDI. mp25 and p2IDI is to be transformed within the JCL1613 host, to further employ physical (UV) mutation method to improve the strain, utilizing 50mL shake flask for screening. A mutated strain can be obtained after JCL1613 goes through UV mutation screening, this mutated strain is called M2H, and it may produce a higher quantity of carotenoids.
(b) creating plasmids for the production of zeaxanthin, β-cryptoxanthin, canthaxanthin and astaxanthin, such as FIG. 5A to FIG 8B: wherein the genes crtY and crtZ are cloned within *Erwinia uredovora*; crtW genes are cloned within *Brevundimonas sp*.; and genes for β-carotene hydroxyl enzymes with N terminal defects (code bh4; crtZde genes) are cloned within Arabidopsis thaliana. The crtY genes are constructed in mp25, forming mp25y or mpy; crtZ genes are constructed in mp25y, forming mp25yz; crtZ genes are constructed in p2IDI, forming pzI6; crtW genes are constructed in p2IDI, forming w-p2IDI; crtZde(bh4) genes are constructed in p2IDI, forming pI12; crtW-idiglnAP2 genes are constructed in pzI6, forming pwizI6, plasmids mp25yz and zI6, mp25y and pI12, mpy and w-p2IDI, mp25yz and pwizI6 are respectively transferred into host M2H, in order to obtain the production strains for producing zeaxanthin, β-cryptoxanthin, canthaxanthin and astaxanthin.
(c) utilizing these carotenoids to produce strains for performing the optimization of high cell density fermentation.
(d) using the centrifugal method in collaboration with spray drying for the recovery of cells.
(e) using solvents or supercritical extraction method to recover and purify these carotenoids to obtain high yield and high purity lycopene, zeaxanthin, β-cryptoxanthin, canthaxanthin and astaxanthin.

After performing the steps described above, the fermentation of lycopene bacteria may reach 1250 mg/L, and the lycopene purity may reach 96% and above after recovery and purification; the fermentation of zeaxanthin bacteria may reach 1020 mg/L, and the zeaxanthin purity may reach 96% and above after recovery and purification; the fermentation of β-cryptoxanthin bacteria may reach 610 mg/L, and the β-cryptoxanthin purity may reach 85% and above after recovery and purification; the fermentation of canthaxanthin bacteria may reach 1200 mg/L, and the canthaxanthin purity may reach 98% and above after recovery and purification; the fermentation of astaxanthin bacteria may reach 650 mg/L, and the astaxanthin purity may reach 90% and above after recovery and purification.

The lycopene bacteria mp25/p2IDI (in *E. coli.* M2H), zeaxanthin bacteria mp25yz/pzI6 (in *E. coli.* M2H), β-cryptoxanthin mp25y/pI12 (in *E. coli.* M2H), canthaxanthin mpy/w-p2IDI (in *E. coli.* M2H) and astaxanthin mp25yz/pwizI6 (in *E. coli.* M2H) used for production in the present invention have been deposited into the Culture Collection Centre within the Food Industry Research and Development Institute in Taiwan on the 29th of April 2008, in compliance with the Taiwan Patent Law Article 30, and the deposit numbers are BCRC940542, BCRC940543, BCRC940540, BCRC940541 and BCRC940539 respectively.

### BRIEF DESCRIPTION OF THE DRAWINGS

The foregoing aspects, and other features and advantages of the present invention will become more apparent by reference to the following detailed descriptions when taken in conjunction with the accompanying drawings, wherein:
Fig. 1 illustrates the biosynthetic pathway diagram of the carotenoids in *E. coli.* according to the present invention;
Fig. 2 illustrates the flow chart for the process of obtaining biosynthesized carotenoids from mutated bacteria or natural bacteria according to the present invention;
Fig. 3 illustrates the yield comparison diagram for different hosts according to the present invention;
Figs. 4A to 4B illustrate the plasmid diagrams for lycopene production according to the present invention;
Figs. 5A to 5B illustrate the plasmid diagrams for zeaxanthin production according to the present invention;
Figs. 6A to 6B illustrate the plasmid diagrams for β-cryptoxanthin production according to the present invention;
Figs. 7A to 7B illustrate the plasmid diagrams for canthaxanthin production according to the present invention;
Figs. 8A to 8B illustrate the plasmid diagrams for astaxanthin production according to the present invention;
Fig. 9 illustrates HPLC diagram for lycopene after fermentation and purification according to the present invention;
Fig. 10 illustrates HPLC diagram for zeaxanthin after fermentation and purification according to the present invention;
Fig. 11 illustrates HPLC diagram for β-cryptoxanthin after fermentation and purification according to the present invention;
Fig. 12 illustrates HPLC diagram for canthaxanthin after fermentation and purification according to the present invention;
Fig. 13 illustrates HPLC diagram for astaxanthin after fermentation and purification according to the present invention.

### DESCRIPTION OF THE PREFERRED EMBODIMENT

In the following description, numerous specific details are provided in order to give a thorough understanding of the embodiments of the invention. The present invention is described in detail below, along with the preferred embodiments and accompanying drawings, it should be recognized that all the preferred embodiments are for the purpose of illustration only, and not for the purpose of limiting the present invention. Those skilled in the relevant art will recognize, however, that the invention may be practiced without one or more of the specific details, or with other methods, components, materials, etc.

The present invention discloses a process for the rapid mass production of different carotenoids through the utilization of bacteria. The present invention utilizes the improved bacteria via UV mutation, and the improved bacteria are suitable hosts for *E. coli* producing high yield carotenoids. The process of producing carotenoids from *E. coli* utilizes the UV mutated, improved bacteria host in order to create genetically engineered bacteria that are able to produce carotenoids and are suitable for high cell density fermentation, then the genetically engineered bacteria are mass produced via high cell density fermentation followed by recovery and purification process in order to obtain high content carotenoids or different kinds of high purity carotenoid crystals.

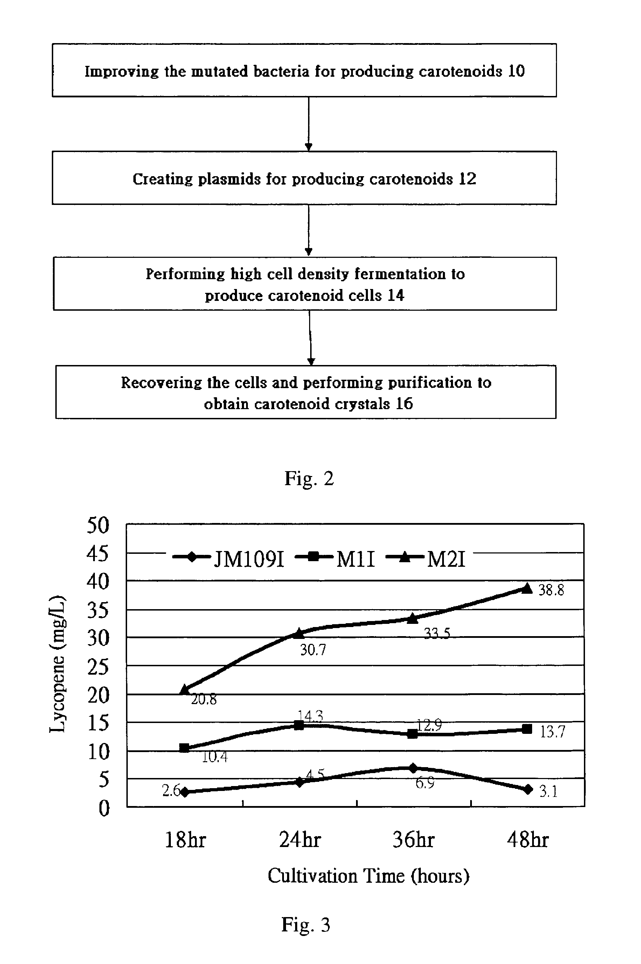

The present invention discloses a process for obtaining biosynthesized carotenoids via mutated bacteria, please refer to Fig. 2 for the process flow chart of the present invention, the process includes: step 10, screening for high yield biosynthesized carotenoid bacteria; step 12, creating carotenoid plasmids; step 14, carrying out high cell density fermentation to produce carotenoid cells; step 16, recovering the cells and performing purification to obtain carotenoid crystals.

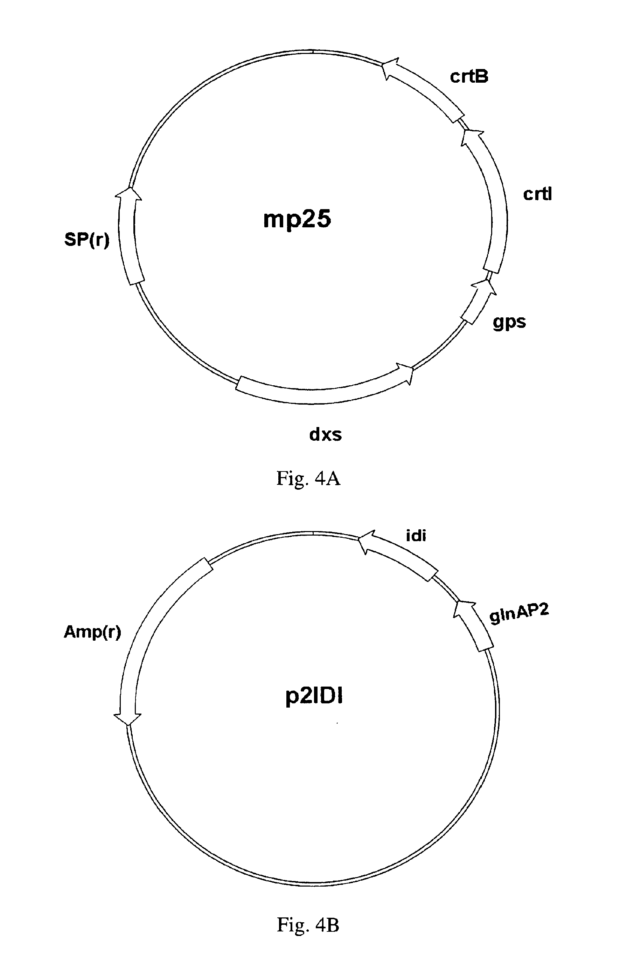

The steps involved in the method disclosed by the present invention are described in detail below:
(1) Step 10, screening for high yield biosynthesized carotenoid bacteria: i.e. screening the host for genetically engineered bacteria. Using lycopene as an index, screening the host for high carotenoid yields, please refer to Fig. 3. Firstly, the patented strain BCRC940321 for producing lycopene has been purchased from the Food Research Institute, and the host of this patented strain, JCL1613 is purified. The genes for biosynthesizing lycopene dxs, gps, crtB and crtI are created in plasmid PCL1920, this plasmid with multiple genes is called mp25. In addition, the patented strain BCRC940323 has been purchased from the Food Research Institute, wherein the plasmid with idi and glnAP2 genes is called p2IDI. mp25 and p2IDI is transformed in the JCL1613 host, physical (UV) mutation method is further utilized to improve the strain, then selecting the portion with 99% death rate, and use the 50 mL shake flask incubation to perform the screening. Through the screening of JCL1613 by ultraviolet (UV) light mutation, a second generation strain called M2H is obtained, and this strain is able to produce a higher yield of lycopene and carotenoids.
(2) Step 12, creating carotenoid plasmids: i.e. creating the genetically engineered production bacteria for producing related carotenoids. Firstly, crty and crtz genes are cloned from *Erwinia uredovora*, crtw gene is cloned from *Brevundimonas sp*. and β-carotene hydroxyl enzymes with N terminal defects (bh4 genes) genes are cloned from Arabidopsis thaliana. By constructing these genes in suitable plasmids, and transformed in *E. coli* respectively, they may be able to biosynthesize genetic recombination strains such as lycopene, zeaxanthin, β-cryptoxanthin, canthaxanthin and astaxanthin etc., please refer Fig. 4A to Fig. 8B for the related plasmids diagrams of each production strain.
(3) Step 14, carrying out high cell density fermentation to produce carotenoid cells: i.e. the fermentation of high cell density bacteria to produce the related carotenoids. After hundreds of researches and experiments regarding fermentation conditions have been carried out, the optimum fermentation conditions for the production bacteria that have been improved through genetic recombination are listed below:
   A: each liter of seed strain medium include 10 grams of yeast extract, 10 grams of peptone and 10 grams of glycerol;
   B: each liter of batch medium include 36 grams of yeast extract, 10.7 grams of dibasic potassium phosphate, 5.4 grams of monobasic potassium phosphate, 10 grams of glycerol, antibiotics Ampicillin (100 ppm) and Streptomycin (200 ppm);
   C: each liter of feeding medium include 60 grams of yeast extract, 90 grams of mixed amino acid powder, 10 grams of magnesium sulfate, 60 grams of monosodium glutamate (MSG), 650 grams of glycerol, antibiotics Ampicillin (200 ppm) and Streptomycin (400 ppm);
   Cultivation temperature of 28-32□; ventilation of 0.3-1.0 vvm; after approximately 96 hours of high cell density fermentation, the dry weight of the bacteria may reach 75-100 grams per liter, wherein the cell weight of lycopene content in the lycopene production strain is roughly 10-15 mg/g, the cell weight of zeaxanthin content in the zeaxanthin production strain is roughly 8-12 mg/g, the cell weight of β-cryptoxanthin content in the β-cryptoxanthin production strain is roughly 5-10 mg/g, the cell weight of canthaxanthin content in the canthaxanthin production strain is roughly 10-20 mg/g, and the cell weight of astaxanthin content in the astaxanthin production strain is roughly 5-10 mg/g. In order to obtain higher yields and higher purity products, minor parameter adjustments during the high cell density fermentation process to suit different production strains may be carried out.
(4) Step 16, recovering the cells and performing purification to obtain carotenoid crystals: utilizing centrifugal method with spray drying to recover cells with carotenoid contents, and using the solvent purification method to extract high purity and high yield carotenoid crystals. The detailed steps are as follows: adding extraction solvent to extract cells with carotenoids; removing the solid state portion of carotenoid cells, and filtering the liquid state portion of carotenoid cells; condensing the carotenoid cells to obtain a crystalline substance; adding solvent to dissolve impurities so the crystalline substance suspends evenly, separating crystalline substance and impurities via filtering; performing drying process on the crystalline substance to obtain high purity carotenoids. The extraction solvent includes dichloromethane and acetone, and solvent includes methane and alcohol.

### Comparisons between High Yield Gene Recombination Hosts

At first, plasmids with biosynthesized lycopene genes are transformed within host JCL1613, creating a strain M1I that is able to produce lycopene. M1I uses the Luria Broth (LB) medium to process overnight cultivation, the bacterial broth is irradiated under UV for 10 minutes (death rate of 99%) and placed in the dark room for 1 hour, the bacterial broth, after being diluted under an appropriate ratio is applied onto the culture plate comprising antibiotics, it is then placed in the incubator at 370 for 48 hours, perform a preliminary screen for the bacterial colony with the redder color, and shake culture for 24 hours at 37□ in the LB medium comprising 1% glycerol, then use OD600 to measure the cell biomass, and utilize acetone to extract lycopene from the cells and measure the content, the results have shown that the lycopene content in this cell is higher than M1I, thus the strain that has been improved by ultraviolet (UV) light is named M2I, and its host is known as M2H. At the same time, plasmids with biosynthesized lycopene genes are transformed within hosts JM109, JCL1613 and M2H (i.e. strains that may produce lycopene JM1091, M1I and M2I), shake culture for 24 hours at 37□ in the LB medium comprising 1% glycerol, the results are shown in Fig. 3, the lycopene yield of M2I is 38.8 mg/L (9.8 mg/g dry weight), which is higher than M1I, it is 3 times more than M1I and 13 times more than JM109.

### Embodiment 1 - Lycopene

### High Cell Density Fermentation

Lycopene seed strain (M2I) is connected into the 50 ml/250 ml shake flask via -80□ holding tube, and placed in an incubator at 32□, 150 rpm for activation. After 8 hours, take out the shake flask and reconnect the seed strain to a 2 L/5 L fermentor, and continue to culture at a temperature of 30□, mixing speed of 400 rpm and ventilation of 2 L/min. When the bacteria count/OD600 in the fermentor reaches 15∼25, start to add feeding medium, continue to culture, and control the pH value to stay between 6.8∼7.2. The total fermentation time takes 96 hours and the yield is 1250 mg/L (HPLC purity is 96%).

### High Cell Density Fermentation and Recovery Purification

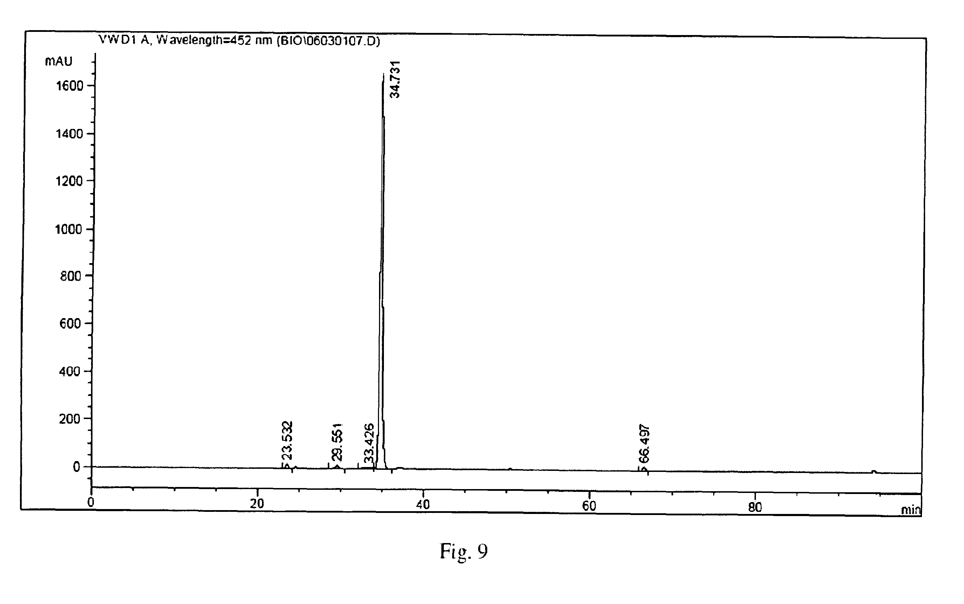

Lycopene seed strain is connected into the 50 ml/250 ml shake flask via -80□ holding tube, and placed in an incubator at 32□, 150 rpm for activation. After 8 hours, take out the shake flask and reconnect the seed strain to a 4 L/5 L fermentor to continue culturing, the activation conditions are controlled at a temperature of 30□, mixing speed of 400 rpm and ventilation of 2 L/min for culturing seed strain (II). After culturing seed strain (II) for approximately 14 hours (OD600 is 22.8 at this moment), connect the bacterial broth to the 400 L/1 KL fermentor to continue culturing, the fermentation conditions are controlled at a temperature of 300, mixing speed of 120 rpm and ventilation of 400 L/min for culturing; when the bacteria count/OD600 reaches 14.5, start to add feeding medium to culture, and control the pH value to stay between 6.8∼7.5. The total fermentation time takes 96 hours and the yield is 880 mg/L (HPLC purity is 96%). After washing, spray drying and using the solvent to extract and purify the fermentation broth, lycopene crystalline powder with the purity of 97% (refer to Fig. 9) and weighing 320 g may be obtained.

### Embodiment 2 - Zeaxanthin

### High Cell Density Fermentation

Zeaxanthin seed strain is connected into the 50 ml/250 ml shake flask via -800 holding tube, and placed in an incubator at 32□, 150 rpm for activation. After 8 hours, take out the shake flask and reconnect the seed strain to a 2 L/5 L fermentor, and continue to culture at a temperature of 30□, mixing speed of 400 rpm and ventilation of 2 L/min. When the bacteria count/OD600 in the fermentor reaches 20, start to add feeding medium, continue to culture, and control the pH value to stay between 7.0∼7.5. The total fermentation time takes 100 hours and the yield is 1020 mg/L (HPLC purity is 82%).

### High Cell Density Fermentation and Recovery Purification

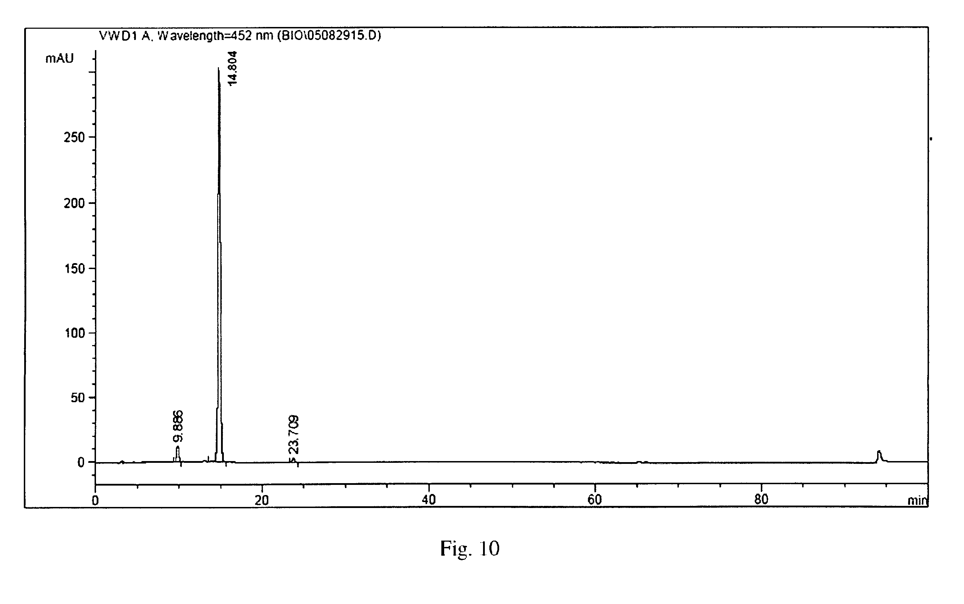

Zeaxanthin seed strain is connected into the 50 ml/250 ml shake flask via -80□ holding tube, and placed in an incubator at 320, 150 rpm for activation. After 10 hours, take out the shake flask and reconnect the seed strain to a 4 L/5 L fermentor to continue culturing, the activation conditions are controlled at a temperature of 30□, mixing speed of 400 rpm and ventilation of 2 L/min for culturing seed strain (II). After culturing seed strain (II) for approximately 14 hours (where OD600 is 25 at this moment), connect the bacterial broth to the 400 L/1 KL fermentor to continue culturing, the fermentation conditions are controlled at a temperature of 30□, mixing speed of 120 rpm and ventilation of 400 L/min for culturing; when the bacteria count/OD600 reaches 15, start to add feeding medium to culture, and control the pH value to stay between 7.0∼7.5. The total fermentation time takes 98 hours and the yield is 850 mg/L (HPLC purity is 81 %). After washing, spray drying and using the solvent to extract and purify the fermentation broth, zeaxanthin crystalline powder with the purity of 98% (refer to Fig. 10) and weighing 250 g may be obtained.

### Embodiment 3 - β-cryptoxanthin

### High Cell Density Fermentation

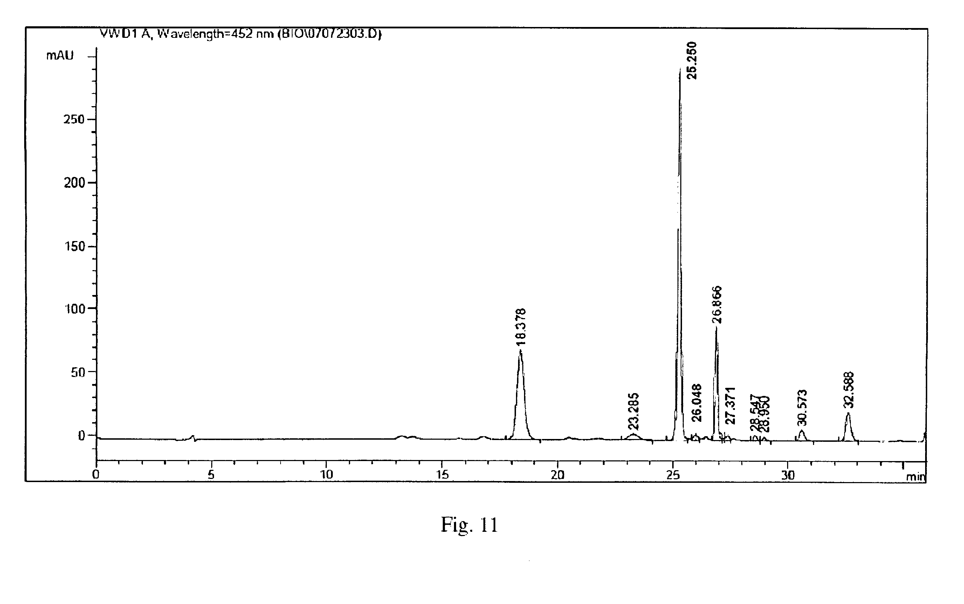

β-cryptoxanthin seed strain is connected into the 50 ml/250 ml shake flask via -800 holding tube, and placed in an incubator at 32□, 150 rpm for activation. After 8 hours, take out the shake flask and reconnect the seed strain to a 2 L/5 L fermentor, and continue to culture at a temperature of 30□, mixing speed of 400 rpm and ventilation of 2 L/min. When the bacteria count/OD600 in the fermentor reaches 20, start to add feeding medium, continue to culture, and control the pH value to stay between 7.0∼7.5. The total fermentation time takes 96 hours and the yield is 610 mg/L (HPLC purity is 68.1 %), refer to Fig. 11.

### Embodiment 4 - Canthaxanthin

### High Cell Density Fermentation

Canthaxanthin seed strain is connected into the 50 ml/250 ml shake flask via -80□ holding tube, and placed in an incubator at 32□, 150 rpm for activation. After 8 hours, take out the shake flask and reconnect the seed strain to a 2 L/5 L fermentor, and continue to culture at a temperature of 300, mixing speed of 400 rpm and ventilation of 2 L/min. When the bacteria count/OD600 in the fermentor reaches 25.3, start to add feeding medium, continue to culture, and control the pH value to stay between 6.8∼7.2. The total fermentation time takes 96 hours and the yield is 1178.6 mg/L (HPLC purity is 78.1%).

### High Cell Density Fermentation and Recovery Purification

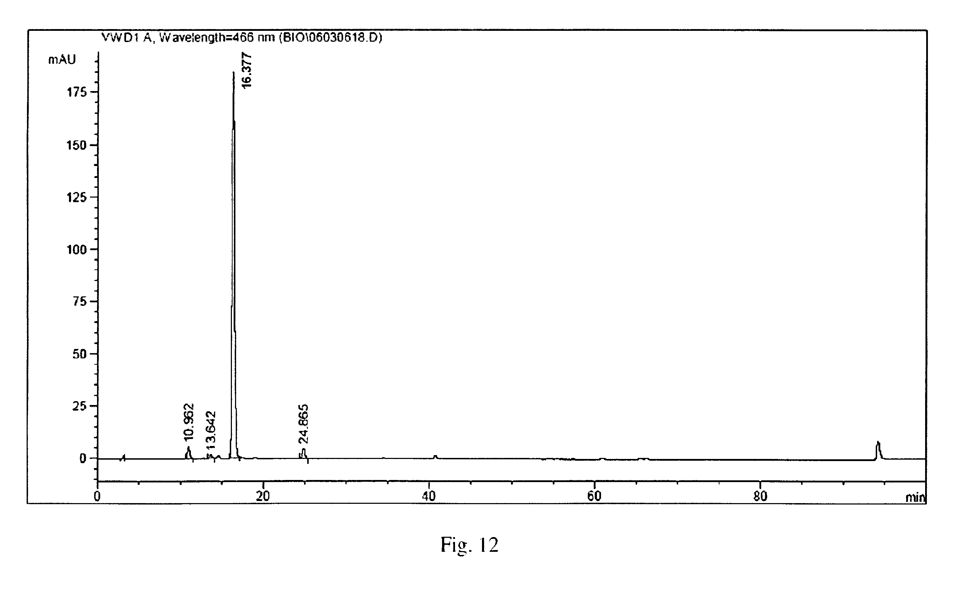

Canthaxanthin seed strain (I) is connected into the 50 ml/250 ml shake flask via -80□ holding tube, and placed in an incubator at 32□, 150 rpm for activation. After 8 hours, take out the shake flask and reconnect the seed strain to a 4 L/5 L fermentor to continue culturing, the activation conditions are controlled at a temperature of 30□, mixing speed of 400 rpm and ventilation of 2 L/min for culturing seed strain (II). After culturing seed strain (II) for approximately 14 hours (where OD600 is 22.8 at this moment), connect the bacterial broth to the 400 L/1 KL fermentor to continue culturing, the fermentation conditions are controlled at a temperature of 30□, mixing speed of 120 rpm and ventilation of 400 L/min for culturing; when the bacteria count/OD600 reaches 14.5, start to add feeding medium to culture, and control the pH value to stay between 6.8∼7.5. The total fermentation time takes 69 hours and the yield is 866.3 mg/L (HPLC purity is 68.8%). After washing and spray drying of the fermentation broth, dried bacterial powder with water content of 8.2% and canthaxanthin content of 5.94 mg/g may be obtained. Then, through solvent extraction, canthaxanthin crystalline powder with the purity of 98% and weighing 220 g may be obtained, refer to Fig. 12.

### Embodiment 5 - Astaxanthin

### Creating and Culturing of Production Strains

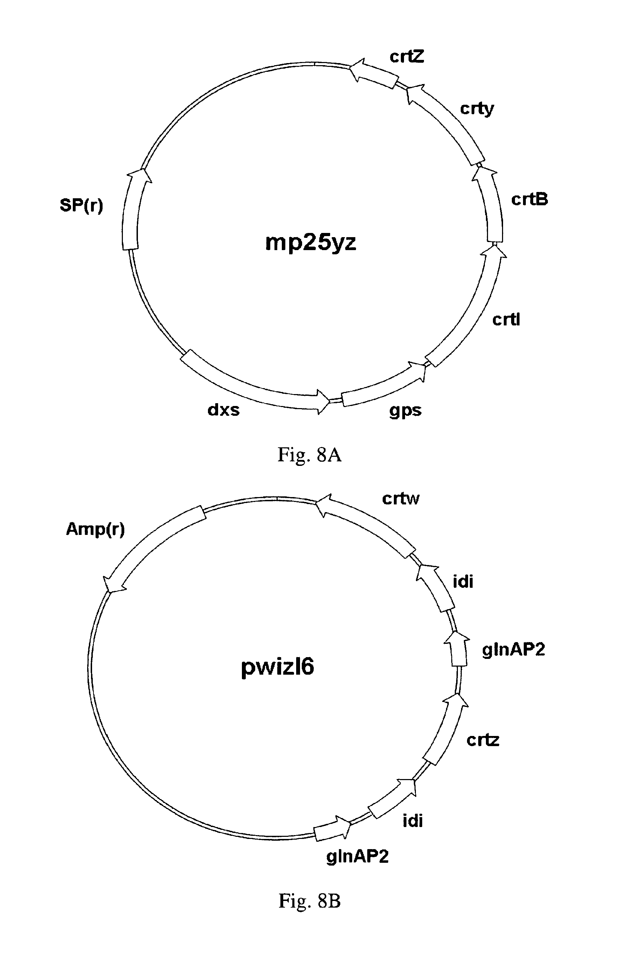

Utilizing M2H as the host, further create strains that may be biosynthesized as astaxanthin. crty and crtz genes are cloned from *Erwinia uredovora*, crty is first created at the sac I position of mp25, and crtz is created at the apaI position, this new plasmid is called mp25yz (Fig. 8A), at the same time crtz gene is connected into the HindIII position of p2IDI, this new plasmid is called pzI6; on the other hand, crtw gene is cloned from *Brevundimonas aurantiaca*, this gene is created in pzI6 along with idi and glnAP2, this new plasmid is called pwizI6 (Fig. 8B); plasmids mp25yz and pwizI6 are transformed in the M2H host together, forming a new bacteria Ast6 that is able to produce astaxanthin. Ast6 is cultured in the LB comprising AP and SP at 32□ and shake culture for 24 hours, resulting in an astaxanthin content of 3.7 mg/g DCW and HPLC purity of 59%.

### High Cell Density Fermentation

Astaxanthin seed strain is connected into the 50 ml/250 ml shake flask via -80□ holding tube, and placed in an incubator at 32□, 150 rpm for activation. After 8 hours, take out the shake flask and reconnect the seed strain to a 2 L/5 L fermentor, and continue to culture at a temperature of 30□, mixing speed of 400 rpm and ventilation of 2 L/min. When the bacteria count/OD600 in the fermentor reaches 15, start to add feeding medium, continue to culture, and control the pH value to stay between 6.8∼7.2. The total fermentation time takes 99 hours and the yield is 1100 mg/L (HPLC purity is 40%).

### High Cell Density Fermentation and Recovery Purification

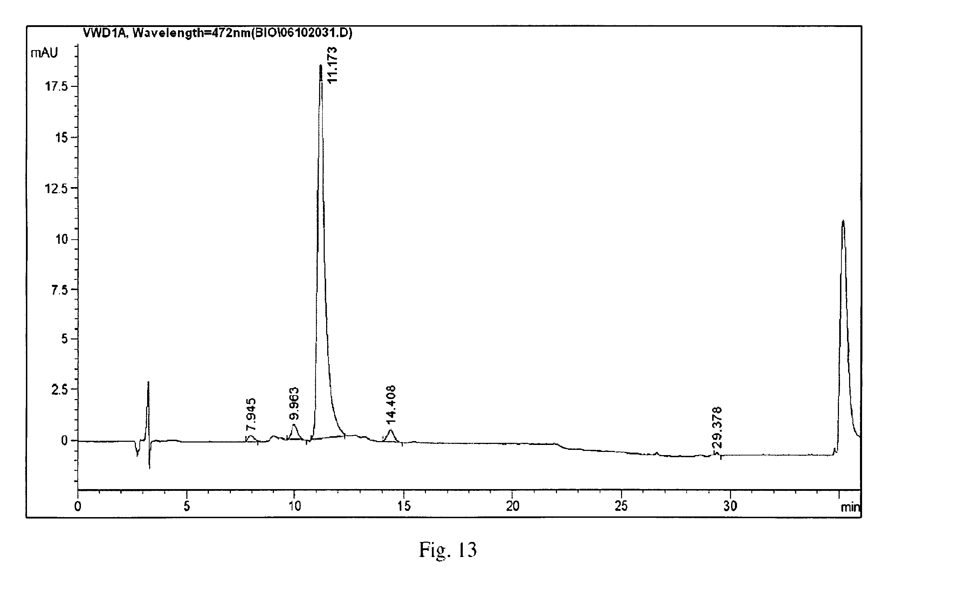

Astaxanthin seed strain is connected into the 50 ml/250 ml shake flask via -800 holding tube, and placed in an incubator at 32□, 150 rpm for activation. After 8 hours, take out the shake flask and reconnect the seed strain to a 4 L/5 L fermentor to continue culturing, the activation conditions are controlled at a temperature of 30□, mixing speed of 400 rpm and ventilation of 2 L/min for culturing seed strain (II). After culturing seed strain (II) for approximately 14 hours (where OD600 is 24.8 at this moment), connect the bacterial broth to the 350 L/1 KL fermentor to continue culturing, the fermentation conditions are controlled at a temperature of 30□, mixing speed of 120 rpm and ventilation of 400 L/min for culturing; when the bacteria count/OD600 reaches 13.1, start to add feeding medium to culture, and the control conditions is that the pH value needs to be less than or equal to 7.1, when the pH value is greater than 7.1, the system automatically adds the feeding medium into the fermentor. The total fermentation time takes 100 hours and the yield is 411.1 mg/L (HPLC purity is 65.5%). After washing and spray drying of the fermentation broth, dried bacterial powder with water content of 11% and astaxanthin content of 5.24 mg/g (HPLC purity of 52.3%) may be obtained. Then, through solvent extraction, astaxanthin crystalline powder with purity greater than 85% may be obtained, refer to Fig. 13.

### Deposit of Microorganisms

The lycopene bacteria mp25/p2IDI (in *E. coli.* M2H), zeaxanthin bacteria mp25yz/pzI6 (in *E. coli.* M2H), β-cryptoxanthin mp25y/pI12 (in *E. coli.* M2H), canthaxanthin mpy/w-p2IDI (in *E. coli.* M2H) and astaxanthin mp25yz/pwizI6 (in *E. coli.* M2H) used for production in the present invention have been deposited into the Culture Collection Centre within the Food Industry Research and Development Institute in Taiwan on the 29th of April 2008, in compliance with the Taiwan Patent Law Article 30, and the deposit numbers are BCRC940542, BCRC940543, BCRC940540, BCRC940541 and BCRC940539 respectively.

The advantages of the present invention are that it provides a process of obtaining biosynthesized carotenoids from transformed bacteria, provides *E. coli* that has been modified through genetic engineering processes and is able to produce high content carotenoids, and provides high density culture methods and recovery purification methods for carotenoids. The present invention is able to make use of the improvements in bacteria and constructions of gene combinations and plasmids, utilizing high cell density fermentation production methods to biosynthesize carotenoids, recover and dry so that high content carotenoids may be obtained, thus achieving the goal of effective and rapid production of the required carotenoids via the biological cell factory mode.

An advantage of the present invention is that it utilizes metabolic engineering control method as the basis for proceeding with conventional bacteria improvement, obtains a higher yield lycopene production strain, and further develops this strain into a biological platform for producing large volumes of carotenoids. By building the biosynthetic pathway of carotenoids onto this biological platform, bacteria for producing various types of carotenoids may be formed, such as zeaxanthin, β-cryptoxanthin, canthaxanthin and astaxanthin, etc., accompanied by high cell density fermentation technology, and recovery purification technology. Therefore, the present invention may be able to overcome the existing biotechnology problems for producing carotenoids, and achieve the target of commercial production

As is understood by a person skilled in the art, the foregoing preferred embodiments of the present invention are illustrative of the present invention rather than limiting the present invention. The present invention is intended to cover various modifications and similar arrangements included within the sprit and scope of the appended claims, the scope of which should be accorded the broadest interpretation so as to encompass all such modifications and similar structures.

## Claims

1. A strain of *Escherichia coli* (*E. coli* M2H) for biosynthesis of high yield carotenoids is a mutated strain obtained from the steps comprising:
purifying the host strain JCL1613 of strain pCW9/P2IDIi;
creating plasmids for producing carotenoid genes;
transforming said plasmids for producing carotenoid genes into said strain JCL1613;
utilizing physical method (UV) to mutate and improve said strain JCL1613;
selecting the portion with roughly 99% death rate;
utilizing 50mL shake flask incubation; and
performing ultraviolet (UV) mutation screening to obtain mutated strain of *E. coli* M2H

2. The *E. coli* M2H for biosynthesis of high yield carotenoids of Claim 1, wherein said carotenoids include lycopene, zeaxanthin, β-cryptoxanthin, canthaxanthin or astaxanthin.

3. The *E. coli* M2H for biosynthesis of high yield carotenoids of Claim 1, wherein said plasmids for carotenoid genes include plasmids for biosynthesizing lycopene mp25 and p2IDI, plasmids for biosynthesizing zeaxanthin mp25yz and pzI6, plasmids for biosynthesizing β-cryptoxanthin mp25y and pI12, plasmids for biosynthesizing canthaxanthin mpy and w-p2IDI and plasmids for biosynthesizing astaxanthin mp25yz and pwiZI6.

4. The *E. coli* M2H for biosynthesis of high yield carotenoids of Claim 3, wherein said plasmid mp25 includes dxs, gps, crtB and crtI genes, said plasmid mp25y includes mp25 and crtY genes, said plasmid mp25yz includes mp25y and crtZ genes.

5. The *E. coli* M2H for biosynthesis of high yield carotenoids of Claim 3, wherein said plasmid p2IDI includes idi and glnAP2 promoter, said plasmid pzI6 includes p2IDI and crtZ genes, said plasmid pI12 includes p2IDI and crtZde(bh4) genes, said plasmid w-p2IDI includes p2IDI and crtW genes, said plasmid pwizI6 includes p2IDI, crtZ and crtW-idi-glnAP2 genes.
